# EUROPEAN PATENT SPECIFICATION

(11) **EP 2 531 854 B1**
(45) Date of publication and mention of the grant of the patent: **19.07.2017**
(21) Application number: 11702696.3
(22) Date of filing: 04.02.2011
(51) Int. Cl.: G01N 33/574, G01N 33/68

(54) **CANCER PROGNOSIS ASSAY**
KREBSPROGNOSE-ASSAY
DOSAGE À VALEUR PRONOSTIQUE UTILISÉ EN CANCÉROLOGIE

(30) Priority: 05.02.2010 GB 201001946
(43) Date of publication of application: 12.12.2012
(73) Proprietor: The Binding Site Group Limited, Birmingham, West Midlands B15 1QT (GB)
(72) Inventor: BRADWELL, Arthur, Birmingham West Midlands B14 4RT (GB); MEAD, Graham, Birmingham West Midlands B14 4RT (GB)
(74) Representative: Elsy, David
(86) International application number: PCT/GB2011/050193
(87) International publication number: WO 2011/095818

(56) References cited:
- EP-A1- 1 870 710
- WO-A1-2006/079816
- BRADWELL A: "Clinical applications of serum free light chain immunoassays", CLINICAL LABORATORY INTERNATIONAL, PAN EUROPEAN PUBLISHING, BRUSSELS, BE, 1 November 2003 (2003-11-01), XP002378158,
- MARSHALL G ET AL: "Borderline high serum free light chain [kappa]/[lambda] ratios are seen not only in dialysis patients but also in non-dialysis-dependent renal impairment and inflammatory states", AMERICAN JOURNAL OF CLINICAL PATHOLOGY 2009 AMERICAN SOCIETY OF CLINICAL PATHOLOGISTS USA LNKD- DOI:10.1309/AJCP8VOT5TVLAQBQ, vol. 132, no. 2, August 2009 (2009-08), page 309, XP002625348, ISSN: 0002-9173

## Description

The invention relates to a method of prognosis of a subject with a cancer, identifying a subject having a greater risk of having an undiagnosed cancer and/or identifying a subject at risk of developing a cancer. The cancer is typically a proinflammmatory cancer, such as a lung, colo-rectal, small bowel, oesophageal or pancreatic (LCBOP) cancer.

The Applicants have for many years studied free light chains as a way of assaying for a wide-range of monoclonal gammopathies in patients. The use of such free light chains in diagnosis is reviewed in detail in the book "Serum Free Light Chain Analysis, Fifth Edition (2008) A.R. Bradwell et al, ISBN 0704427028".

Antibodies comprise heavy chains and light chains. They usually have a two-fold symmetry and are composed of two identical heavy chains and two identical light chains, each containing variable and constant region domains. The variable domains of each light-chain/heavy-chain pair combine to form an antigen-binding site, so that both chains contribute to the antigen-binding specificity of the antibody molecule. Light chains are of two types, κ and λ and any given antibody molecule is produced with either light chain but never both. There are approximately twice as many κ as λ molecules produced in humans, but this is different in some mammals. Usually the light chains are attached to heavy chains. However, some unattached "free light chains" are detectable in the serum or urine of individuals. Free light chains may be specifically identified by raising antibodies against the surface of the free light chain that is normally hidden by the binding of the light chain to the heavy chain. In free light chains (FLC) this surface is exposed, allowing it to be detected immunologically. Commercially available kits for the detection of κ or λ free light chains include, for example, "Freelite™", manufactured by The Binding Site Limited, Birmingham, United Kingdom. The Applicants have previously identified that determining the amount of free κ/free λ ratios, aids the diagnosis of monoclonal gammopathies in patients. It has been used, for example, as an aid in the diagnosis of intact immunoglobulin multiple myeloma (MM), light chain MM, non-secretory MM, AL amyloidosis, light chain deposition disease, smouldering MM, plasmacytoma and MGUS (monoclonal gammopathies of undetermined significance). Detection of FLC has also been used, for example, as an aid to the diagnosis of other B-cell dyscrasia and indeed as an alternative to urinary Bence Jones protein analysis for the diagnosis of monoclonal gammopathies in general.

Conventionally, an increase in one of the λ or κ light chains and a consequently abnormal ratio is looked for. For example, multiple myelomas result from the monoclonal multiplication of a malignant plasma cell, resulting in an increase in a single type of cell producing a single type of immunoglobulin. This results in an increase in the amount of free light chain, either λ or κ, observed within an individual. This increase in concentration may be determined, and usually the ratio of the free κ to free λ is determined and compared with the normal range. This aids in the diagnosis of monoclonal disease. Moreover, the free light chain assays may also be used for the following of treatment of the disease in patients. Prognosis of, for example, patients after treatment for AL amyloidosis may be carried out.

Katzman et al (Clin. Chem. (2002); 48(9): 1437-1944) discuss serum reference intervals and diagnostic ranges for free κ and free λ immunoglobulins in the diagnosis of monoclonal gammopathies. Individuals from 21-90 years of age were studied by immunoassay and compared to results obtained by immunofixation to optimise the immunoassay for the detection of monoclonal free light chains (FLC) in individuals with B-cell dyscrasia.
The amount of κ and λ FLC and the κ/λ ratios were recorded allowing a reference interval to be determined for the detection of B-cell dyscrasias.

The Applicants have now identified that assaying for FLC and especially total FLC can be used in a method of prognosis of a subject with a cancer, identifying a subject having a greater risk of having an undiagnosed cancer and/or identifying a subject at risk of developing a cancer. They have found that FLC concentration is statistically significantly linked to risk of death in subjects from such cancers and to be indicative of the presence of such a cancer.

The concentration of FLC in serum from individuals that are apparently healthy is influenced by the ability to some extent of the individual's kidneys to filter and excrete FLC. In individuals where FLC clearance is restricted, there is an increase in the levels of FLC found in serum. As a consequence, it is now believed that FLC is a good marker of renal function. Because monomeric FLC kappa molecules (25kDa) are of different size to dimeric lambda molecules (50kDa), together they are better markers of glomerular filtration than, for example, creatinine113kDa). However, in contrast to creatinine, production of FLCs may result as a consequence of many diseases, so serum FLCs will typically not be used as a renal function marker, in isolation.

However, markers of B-cell proliferation/activity are important and because B-cells are responsible for making FLCs, this is clinically useful. FLC production is an early indicator of B-cell up-regulation. In this respect it can complement the use of CRP which is a T-cell mediated marker of inflammatory responses.

High FLC concentrations may well be an indication of chronic renal or inflammatory disorders or B-cell dyscrasias. Hence, an abnormal FLC assay result may be a marker of a variety of disorders that currently require several tests in combination. The converse of this, when the FLC assay results are normal, indicates good renal function, no inflammatory conditions and no evidence of B-cell dyscrasia.

The Applicant studied serum samples from patients having various degrees of renal impairment. The causes of death of patients was investigated in comparison with FLC concentration. No association between cancer and renal function was observed, but FLC levels, and especially total FLC levels were observed to be clearly associated with risk of death from cancer. A probability of P<0.033 was observed.

The invention provides a method of prognosis of a subject with a cancer as defined in claim 1.

Typically the subject has not previously been diagnosed with the cancer.

The cancer may be a proinflammatory cancer, for example a lung, colo-rectal, small bowel, oesophageal or pancreatic (LCBOP) cancer

The FLC may be kappa or lambda FLC. However, preferably the total FLC concentration is measured, as detecting kappa FLC or lambda FLC alone may miss, for example abnormally high levels of one or other FLC produced for example monoclonally in the patient.

Total free light chain means the total amount of free kappa plus free lambda light chains in a sample.

Moreover, the individual typically does not have reduced bone marrow function. The individual typically does not have an abnormal λ : κ FLC ratio, typically found in many such diseases.

The term "total free light chains" means the amount of κ and λ free light chains in the sample from the subject.

The sample is typically a sample of serum from the subject. However, whole blood, plasma, urine or other samples of tissue or fluids may also potentially be utilised.

Typically the FLC, such as total FLC, is determined by immunoassay, such as ELISA assays or utilising fluorescently labeled beads, such as Luminex ^{™} beads.

Sandwich assays, for example, use antibodies to detect specific antigens. One or more of the antibodies used in the assay may be labeled with an enzyme capable of converting a substrate into a detectable analyte. Such enzymes include horseradish peroxidase, alkaline phosphatase and other enzymes known in the art. Alternatively, other detectable tags or labels may be used instead of, or together with, the enzymes. These include radioisotopes, a wide range of coloured and fluorescent labels known in the art, including fluorescein, Alexa fluor, Oregon Green, BODIPY, rhodamine red, Cascade Blue, Marina Blue, Pacific Blue, Cascade Yellow, gold; and conjugates such as biotin (available from, for example, Invitrogen Ltd, United Kingdom). Dye sols, chemiluminescent labels, metallic sols or coloured latex may also be used. One or more of these labels may be used in the ELISA assays according to the various inventions described herein or alternatively in the other assays, labeled antibodies or kits described herein.

The construction of sandwich-type assays is itself well known in the art. For example, a "capture antibody" specific for the FLC is immobilised on a substrate. The "capture antibody" may be immobilised onto the substrate by methods which are well known in the art. FLC in the sample are bound by the "capture antibody" which binds the FLC to the substrate via the "capture antibody".

Unbound immunoglobulins may be washed away.

In ELISA or sandwich assays the presence of bound immunoglobulins may be determined by using a labeled "detecting antibody" specific to a different part of the FLC of interest than the binding antibody.

Flow cytometry may be used to detect the binding of the FLC of interest. This technique is well known in the art for, e.g. cell sorting. However, it can also be used to detect labeled particles, such as beads, and to measure their size. Numerous text books describe flow cytometry, such as Practical Flow Cytometry, 3rd Ed. (1994), H. Shapiro, Alan R. Liss, New York, and Flow Cytometry, First Principles (2nd Ed.) 2001, A.L. Given, Wiley Liss.

One of the binding antibodies, such as the antibody specific for FLC, is bound to a bead, such as a polystyrene or latex bead. The beads are mixed with the sample and the second detecting antibody. The detecting antibody is preferably labeled with a detectable label, which binds the FLC to be detected in the sample. This results in a labeled bead when the FLC to be assayed is present.

Other antibodies specific for other analytes described herein may also be used to allow the detection of those analytes.

Labeled beads may then be detected via flow cytometry. Different labels, such as different fluorescent labels may be used for, for example, the anti- free λ and anti- free κ antibodies. Other antibodies specific for other analytes, such as cancer-specific antigens, described herein may also be used in this or other assays described herein to allow the detection of those analytes. This allows the amount of each type of FLC bound to be determined simultaneously or the presence of other analytes to be determined.

Alternatively, or additionally, different sized beads may be used for different antibodies, for example for different marker specific antibodies. Flow cytometry can distinguish between different sized beads and hence can rapidly determine the amount of each FLC or other analyte in a sample.

An alternative method uses the antibodies bound to, for example, fluorescently labeled beads such as commercially available Luminex^{™} beads. Different beads are used with different antibodies. Different beads are labeled with different fluorophore mixtures, thus allowing different analytes to be determined by the fluorescent wavelength. Luminex beads are available from Luminex Corporation, Austin, Texas, United States of America.

Preferably the assay used is a nephelometric or turbidimetric method. Nephelometric and turbidimetric assays for the detection of λ - or κ - FLC are generally known in the art, but not for total FLC assays. They have the best level of sensitivity for the assay. λ and κ FLC concentrations may be separately determined or a single assay for total FLC arrived at. Such an assay contains anti-κ and anti-λ FLC antibodies typically at a 60:40 ratio, but other ratios, such as 50:50 may be used.

Antibodies may also be raised against a mixture of free λ and free κ light chains.

The amount of FLC such as total FLC may be compared to a standard, predetermined value to determine whether the total amount is higher or lower than a normal range of FLC.

As discussed in detail below, the Applicants have identified that higher concentrations of serum FLC are associated with a significant increase in the likelihood of reduced survival in patients with pro-inflammatory cancers. More so than, for example, for people with lower serum FLC levels.

A level of >1.7 mg/L of FLC per unit GFR was associated with an increased risk of death from cancer. Patients with a level above the 90^{th} percentile (6.12mg/L of FLC per unit GFR) had a very significantly increased risk (P<0.005).

Historically, assay kits have been produced for measurement of kappa and lambda FLC separately, to allow the calculation of a ratio. They have been conventionally used in individuals already exhibiting disease symptoms.

Preferably the assay is capable of determining FLC, for example total FLC, in the sample for example from approximately 1 mg/L to 100 mg/L, or 1 mg/L - 80 mg/L. This is expected to detect the serum FLC concentrations in the vast majority of individuals without the requirement for re-assaying samples at a different dilution.

Preferably the method comprises detecting the amount of total free light chain in the sample utilising an immunoassay, for example, by utilising a mixture of anti-free κ light chain and anti-free λ light chain antibodies or fragments thereof. Such antibodies may be in a ratio of 50:50 anti-κ: anti-λ antibodies. Antibodies, or fragments, bound to FLC may be detected directly by using labelled antibodies or fragments, or indirectly using labelled antibodies against the anti-free λ or anti-free κ antibodies.

The antibodies may be polyclonal or monoclonal. Polyclonal may be used because they allow for some variability between light chains of the same type to be detected as they are raised against different parts of the same chain. The production of polyclonal antibodies is described, for example in WO97/17372.

Preferably, the amount of serum FLC, such as total FLC, identified, and found to be significant to show an increased likelihood of overall survival, is below 50 mg/L. A level of <47.4mg/L showed P<0.001.

Individuals with a corrected FLC level above 1.7mg/L per unit GFR (glomerular filtration rate) had a markedly shorter survival. Patients with a level above the 90^{th} percentile (6.12mg/L FLC per unit GFR) had a very significantly increased risk (P<0.005).

Assay kits for FLC, for example for use in the methods of the invention are also described. The kits may detect the amount total FLC in a sample. They may be provided in combination with instructions for use in the methods of the invention.

Use of assay kits are also for use in a method according to the invention are provided, comprising one or more anti-FLC antibodies and one or more anti-LCBOP cancer antigen antibodies. Antigens which are markers are generally known for many of the cancers. Antibodies may be provided for such markers to further identify the cancers.

The assay kits may be adapted to detect an amount of total free light chain (FLC) in a sample below 25 mg/L, most preferably, below 20 mg/L or about, 10 mg/L, below 5 mg/L or 4 mg/L. The calibrator material typically measures the range 1-100mg/L. The assay kit may be, for example, a nephelometric assay kit. Preferably the kit is an immunoassay kit comprising one or more antibodies against FLC. Typically the kit comprises a mixture of anti-K and anti-λ FLC antibodies. Typically a mixture of 50:50 anti-free κ and anti-free λ antibodies are used. The kit may be adapted to detect an amount of 1 - 100 mg/L, or preferably 1 - 80 mg/L total free light chain in a sample.

Fragment of antibodies, such as (Fab)₂ or Fab antibodies, which are capable of binding FLC may also be used.

The antibodies or fragments may be labelled, for example with a label as described above. Labelled anti-immunoglobulin binding antibodies or fragments thereof may be provided to detect anti-free λ or anti-free κ bound to FLC.

The kit may comprise calibrator fluids to allow the assay to be calibrated at the ranges indicated. The calibrator fluids preferably contain predetermined concentrations of FLC, for example 100mg/L to 1mg/L, below 25 mg/L, below 20 mg/L, below 10 mg/L, below 5 mg/L or to 1 mg/L. The kit may also be adapted by optimising the amount of antibody and "blocking" protein coated onto the latex particles and, for example, by optimising concentrations of supplementary reagents such as polyethylene glycol (PEG) concentrations.

The kit may comprise, for example, a plurality of standard controls for the FLC. The standard controls may be used to validate a standard curve for the concentrations of the FLC or other components to be produced. Such standard controls confirm that the previously calibrated standard curves are valid for the reagents and conditions being used. They are typically used at substantially the same time as the assays of samples from subjects. The standards may comprise one or more standards below 20 mg/L for FLC, more preferably below 15 mg/L, below approximately 10 mg/L or below 5 mg/L, in order to allow the assay to detect the lower concentrations of free light chain.

The assay kit may be a nephelometric or turbidimetric kit. It may be an ELISA, flow cytometry, fluorescent, chemiluminescent or bead-type assay or dipstick. Such assays are generally known in the art.

The assay kit may also comprise instructions to be used in the method according to the invention. The instructions may comprise an indication of the concentration of total free light chain considered to be a normal value, below which, or indeed above which, shows an indication of either increased or decreased probability of survival of the individual or an increased likelihood of the cancer being present, for example. Such concentrations may be as defined above.

The invention will now be described by way of example only, with reference to the following figures:
**Figure 1** shows the probability of survival for a study population divided into quintiles on the basis of their total FLC concentrations. The quintile levels were <33.3, 33.4-47.3, 47.4-76.8, 67.9-106.3 and >106.5 mg/L
**Figure 2** is a comparison between the total FLC concentrations obtained using separate, commercially available, anti-free κ and anti-free λ assay kits, compared to a total FLC assay kit using combined anti-λ and anti-κ free light chain antibodies.

### Tumour prognosis

### Background

Deaths due to cancer remain a common cause of death. Early diagnosis and treatment can dramatically improve patient outcomes, making the continued development of accurate screening tests invaluable. Several cancers have a pro-inflammatory nature and it was hypothesised that elevated serum, immunoglobulin, free light chain concentrations might be a useful marker of the presence or ongoing development of one of these tumours. Specific cancers considered were those with significant inflammatory associations (lung, colo-rectal, small bowel, oesophageal, pancreatic; LCBOP).

### Methods

1300 patients with various degrees of renal impairment had serum samples collected ("Baseline") and were then followed up for a period of up to 63 months.
In more detail the patients were recruited from the renal clinics at the University Hospital Birmingham. The patients had a range of renal problems including proteinuria, haematuria, chronic kidney disease (all stages), end stage renal failure (haemodialysis and peritoneal dialysis) and renal transplant recipients.

The tests and assessments made included:
Serum creatinine and an estimated glomerular filtration rate (eGFR).

A corrected level ofFLCs per unit GFR was calculated as follows: total serum FLC concentration (mg/L) was divided by estimated glomerular filtration rate as calculated by the Cockcroft-Gault equation (REF) in mls/min/1.73m². Thus giving a serum total FLC level for the patient, independent of renal function, in mg/L per unit GFR.

### Ref:

Cockcroft DW, Gault MH: Prediction of creatinine clearance from serum creatinine. Nephron 16: 31-41, 1976

Urinary albumin/creatinine ratio.
These are standard tests in the art.

Serum FLC concentrations, both kappa and lambda (Freelite, The Binding Site, Birmingham, UK).
Total, serum FLC concentrations were calculated by adding the values for kappa FLC and lambda FLC.

### Follow-up:

Patients were followed up for time to death and cause of death.

### Results

Kaplan Meier analysis of patient survival demonstrated that patients with higher FLC levels had a reduced survival (P<0.001).

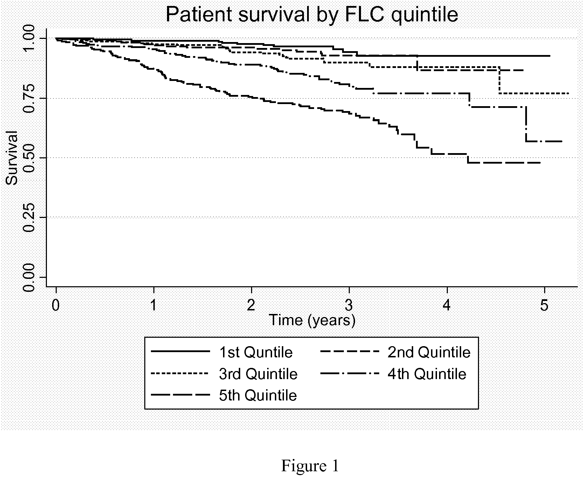

Survival rates are shown in Figure 1

| | | Percentile Group of total FLCs (range mg/L) | | | | | |
|---|---|---|---|---|---|---|---|
| | | 1 (<33.3) | 2 (33.4-47.3) | 3 (47.4-67.8) | 4 (67.9-106.3) | 5 (>106.5) | Total |
| Dead | No | 266 | 262 | 256 | 228 | 181 | 1193 |
| | Yes | 11 | 16 | 23 | 48 | 87 | 185 |
| | Total | 277 | 278 | 279 | 276 | 268 | 1378 |

When serum total FLCs were evaluated independently of renal function a level of >1.7 mg/L of FLC per unit GFR was associated with a significantly reduced survival (P<0.0001):

| | | Percentile Group of total FLCs corrected for GFR (range mg/L per unit GFR) | | | | | |
|---|---|---|---|---|---|---|---|
| | | 1 (<0.5) | 2 (0.5-0.9) | 3 (0.9-1.7) | 4 (1.7-3.6) | 5 (>3.6) | Total |
| Dead | No | 255 | 256 | 245 | 219 | 183 | 1158 |
| | Yes | 9 | 10 | 20 | 46 | 81 | 166 |
| | Total | 264 | 266 | 265 | 265 | 264 | 1324 |

Individuals with a corrected FLC level above the median (1.7mg/L per unit GFR) had a markedly shorter survival

When causes of death were investigated the chances of death secondary to cancers was significantly higher in patients with higher total FLC concentrations

| | | | Percentile Group of total FLCs (range mg/ml) | | | | | |
|---|---|---|---|---|---|---|---|---|
| Cause of death | | | 1 (<33.3) | 2 (33.4-47.3) | 3 (47.4-67.8) | 4 (67.9-106.3) | 5 (>106.5) | Total |
| | Cardiovascular | | 3 | 5 | 5 | 14 | 28 | 55 |
| | Infection | | 3 | 1 | 8 | 11 | 23 | 46 |
| | Renal | | 0 | 0 | 2 | 5 | 12 | 19 |
| | Cancer | | 2 | 2 | 3 | 5 | 10 | 22 |
| | Other | | 2 | 0 | 0 | 4 | 3 | 9 |
| | Unknown | | 0 | 0 | 0 | 0 | 1 | 1 |
| | Total | | 10 | 8 | 18 | 39 | 76 | 152 |

The data is more impressive when the nature of the cancer deaths is identified:
FLC quintile 1: Brain tumour, urinary tract tumour
quintile 2: Colon tumour, urinary tract tumour
quintile 3: 3x urinary tract tumours
quintile 4: breast cancer, small bowel tumour, oesophageal cancer, 2xhaematological tumours
quintile 5: liver, 3x haematological tumours, pancreatic cancer, oesophageal cancer, 2x colon tumours, 2x lung tumours.

Therefore, the cancers known to be more proinflammatory or associated with B-cell proliferation were in the higher FLC quintiles. No association between cancer and renal function was found. In a Kaplan Meier analysis high FLC levels were associated with a shorter time to death from cancers (P<0.023).
Total FLC level clearly associated with risk of death from pro-inflammatory cancers.

### Discussion

The results were gathered in patients with some form of renal impairment but renal function is not believed to have any association with the evolution of LCBOP cancers and this was indicated by the lack of any increased incidence in the people with the worst renal function. On this basis it is logical that elevations of total FLC will be similarly predictive of LCBOP cancer incidence in the general population. It is believed that the test(s) will be more sensitive in the general population where there is no background elevation of FLC concentrations due to reduced renal function.

### Assay Kit

The method according to the invention may utilise the following assay kit. The assay kit quantifies the total free κ plus free λ light chains present within patient samples, for example, in serum. This may be achieved by coating 100 nm carboxyl modified latex particles with a 50:50 blend of anti-free κ and anti-free λ light chain sheep antibody. In the assay exemplified below, the measuring range for the total free light chains is for 1-80 mg/L. However, other measuring ranges could equally be considered.

Anti-free κ and anti-free λ anti sera are produced using techniques generally known in the art, in this particular case in sheep. The general immunisation process is described in WO 97/17372.

Anti-κ and anti-λ antisera were diluted to equal concentrations using phosphate buffered saline (PBS). Those antibodies were combined to produce antisera comprising 50% anti κ antibody and 50% anti λ antibody.

Antibodies were coated onto carboxyl modified latex at a coat load of 10 mg/lot. This was achieved using standard procedures. See, for example, "Microparticle Reagent Optimization: A laboratory reference manual from the authority on microparticles" Eds: Caryl Griffin, Jim Sutor, Bruce Shull. Copyright Seradyn Inc, 1994 (P/N 0347835(1294).

This reference also provides details of optimising the assay kits using polyethylene glycol (PEG).

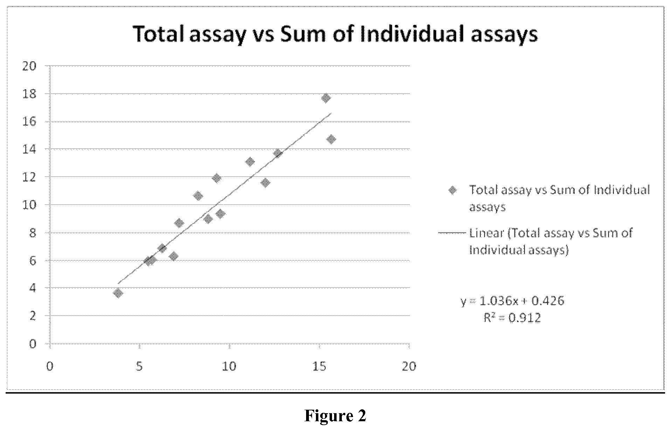

The combined antibodies were compared to results obtained using commercially available κ and λ Freelite™ kits (obtained from the Binding Site Group Limited, Birmingham, United Kingdom). Such Freelite™ kits identify the amount of κ and the amount of λ free light chains in separate assays. The total FLC kits were used to generate curves, which were validated using controlled concentrations. Calibration curves were able to be obtained between 1 and 80 mg/l for total free light chain. In the results table below, results were obtained for κ free light chain (KFLC), λ free light chain (LFLC) and total FLC, using the κ FreeliteTM, λ FreeliteTM and total free light chain assays. These results are shown for 15 different normal serum samples. The results are shown in the table below and in Figure 2 as measured by turbidimetry.

Preliminary results indicate that the principle of using a total free light chain assay based on anti-κ and anti-λ free light chain antibody is viable.

### Results

| | | Result (mg/l) | | | KKLC + LFLC | % diff Total FLC vs (KFLC) + LFLC) |
|---|---|---|---|---|---|---|
| USN | Batch Id | KFLC | LFLC | Total FLC | | |
| 1 | 104 | 3.37 | 3.51 | 6.31 | 6.88 | -8.3% |
| 2 | 151 | 3.42 | 5.39 | 8.99 | 8.81 | 2.0% |
| 3 | 158 | 3.28 | 6.21 | 9.35 | 9.49 | -1.5% |
| 4 | 161 | 2.05 | 3.62 | 6.06 | 5.67 | 6.9% |
| 5 | 179 | 6.83 | 5.84 | 13.71 | 12.67 | 8.2% |
| 6 | 180 | 2.19 | 3.27 | 5.96 | 5.46 | 9.2% |
| 7 | 181 | 2.98 | 5.27 | 10.64 | 8.25 | 29.0% |
| 8 | 182 | 4.72 | 7.26 | 11.6 | 11.98 | -3.2% |
| 9 | 216 | 2.54 | 4.66 | 8.7 | 7.2 | 20.8% |
| 10 | 217 | 3.01 | 3.24 | 6.88 | 6.25 | 10.1% |
| 11 | 219 | 7.12 | 8.53 | 14.73 | 15.65 | -5.9% |
| 12 | 227 | 1.47 | 2.31 | 3.66 | 3.78 | -3.2% |
| 13 | 228 | 8.16 | 7.2 | 17.67 | 15.36 | 15.0% |
| 14 | 229 | 4.51 | 6.61 | 13.1 | 11.12 | 17.8% |
| 15 | 231 | 3.69 | 5.6 | 11.91 | 9.29 | 28.2% |
| | | | | | **Mean** **Diff** | **8.3%** |

## Claims

1. A method of prognosis of a subject with a cancer and/or identifying a subject having a greater risk of having an undiagnosed cancer the method comprising detecting an amount of free light chains (FLC) in a sample of whole blood, plasma or serum from a subject, wherein a higher amount of FLC is associated with decreased survival due to a cancer and/or increased risk of the subject having an undiagnosed cancer wherein the cancer is a lung, colorectal, small bowel, oesophageal or pancreatic (LCBOP) cancer which is not a B-cell associated disease or a myeloma (such as intact immunoglobulin myeloma, light chain myeloma, non-secretory myeloma), an MGUS, AL amyloidosis, Waldenström's macro globulinaemia, Hodgkin's lymphoma, follicular centre cell lymphoma, chroniclymphocytic leukaemia, mantle cell lymphoma, pre-B cell leukaemia or acute lymphoblastic leukaemia, and wherein the amount of free light chains is the amount of total free light chains in the sample.

2. A method according to claim 1, wherein the FLC is determined in a sample of serum from the subject.

3. A method according to claims 1 or 2, wherein the total FLC is determined by immunoassay using anti-free light chain antibodies.

4. A method according to claim 3, wherein the antibodies are a mixture of anti-free κ light chain and anti-free λ light chain antibodies.

5. A method according to claims 1 to 4, wherein the method comprises detecting the amount of FLC by nephelometry or turbidimetry.

6. A method according to any preceding claim wherein the subject has not previously been diagnosed as having cancer and/or does not have symptoms of a B-cell associated disease.

7. Use of an assay kit in a method according to claims 1 to 6, the assay kit comprising one or more anti-FLC antibodies and one or more anti-LCBOP cancer antigen antibodies.

## Patentansprüche

1. Verfahren zur Prognose für einen Probanden mit einem Krebs und/oder zum Identifizieren eines Probanden mit einem erhöhten Risiko für einen undiagnostizierten Krebs, wobei das Verfahren umfasst: Erfassen einer Menge freier Leichtketten (FLC) in einer Vollblut-, Plasma- oder Serumprobe eines Probanden, wobei eine höhere Menge an FLC mit einem aufgrund eines Krebses verringerten Überleben und/oder einem erhöhten Risiko dessen, dass der Proband einen undiagnostizierten Krebs hat, assoziiert ist, wobei der Krebs ein Lungen-, Kolorektal-, Dünndarm-, Speiseröhren- oder Bauchspeicheldrüsen- (LCBOP-) Krebs ist, bei dem es sich nicht um eine B-Zellenassoziierte Erkrankung oder ein Myelom (wie etwa ein Myelom mit intaktem Immunglobulin, Leichtkettenmyelom, nicht-sezernierendes Myelom), eine MGUS, AL-Amyloidose, Makroglobulinämie Waldenström, Hodgkin-Lymphom, Follikel-Zentrumszell-Lymphom, chronische lymphatische Leukämie, Mantelzell-Lymphom, Prä-B-Zell-Leukämie oder akute lymphoblastische Leukämie handelt, und wobei die Menge freier Leichtketten die Menge der gesamten freien Leichtketten in der Probe ist.

2. Verfahren nach Anspruch 1, bei dem die FLC in einer Serumprobe des Probanden bestimmt werden.

3. Verfahren nach Anspruch 1 oder 2, bei dem die Gesamtmenge der FLC durch Immunassay mit Antikörpern gegen freie Leichtketten bestimmt wird.

4. Verfahren nach Anspruch 3, bei dem die Antikörper eine Mischung aus Antikörpern gegen freie κ-Leichtketten und Antikörpern gegen freie λ-Leichtketten sind.

5. Verfahren nach Anspruch 1 bis 4, wobei das Verfahren ein Erfassen der Menge der FLC durch Nephelometrie oder Turbidimetrie umfasst.

6. Verfahren nach einem der vorstehenden Ansprüche, bei dem der Proband zuvor nicht mit Krebs diagnostiziert wurde und/oder keine Symptome einer B-Zellen-assoziierten Erkrankung aufweist.

7. Verwendung eines Assay-Kits in einem Verfahren nach Anspruch 1 bis 6, wobei das Assay-Kit einen oder mehr Anti-FLC-Antikörper und einen oder mehr Anti-LCBOP-Krebs-Antigen-Antikörper umfasst.

## Revendications

1. Procédé permettant d'effectuer un pronostic chez un sujet atteint d'un cancer et/ou d'identifier un sujet ayant un risque plus important d'être atteint d'un cancer non diagnostiqué, ce procédé comprenant des étapes consistant à détecter la quantité de chaînes légères libres (FLC) dans un échantillon de sang total, de plasma ou de sérum d'un sujet, une quantité plus élevée de FLC étant associée à une survie diminuée du fait d'un cancer et/ou à un risque augmenté que le sujet soit atteint d'un cancer non diagnostiqué, le cancer étant un cancer du poumon, un cancer colorectal, un cancer de l'intestin grêle, de l'oesophage ou du pancréas (LCBOP) qui n'est pas une affection associée aux cellules B ou à un myélome (tel qu'un myélome d'immunoglobuline intact, un myélome de chaînes légères, un myélome non sécrétoire) MGUS, AL l'amyloïdose, la macroglobulinémie de Waldenström, le lymphome de Hodgkin, le lymphome des cellules centrales folliculaires, la leucémie chroniclymphocytique, le lymphome du manteau, la leucémie des cellules pré-B ou la leucémie lymphoblastique aigue, et la quantité de chaînes légères libres étant la quantité de chaînes légères libres totale dans l'échantillon.

2. Procédé conforme à la revendication 1,
selon lequel la quantité de FLC est déterminée dans un échantillon de sérum du sujet.

3. Procédé conforme à la revendication 1 ou 2,
selon lequel la quantité de FLC totale est déterminée par un immuno test en utilisant des anticorps anti chaînes légères libres.

4. Procédé conforme à la revendication 3,
selon lequel les anticorps sont un mélange d'anticorps anti chaînes légères κ libres et d'anticorps anti chaîne légère λ libres.

5. Procédé conforme à l'une des revendications 1 à 4,
comprenant une étape de détection de la quantité de FLC par néphélométrie ou turbidimétrie.

6. Procédé conforme à l'une quelconque des revendications précédentes, selon lequel le sujet n'a pas été diagnostiqué auparavent comme atteint d'un cancer et/ou ne présente pas des symptômes d'une affection associée aux cellules B.

7. Utilisation d'un kit de test dans un procédé conforme aux revendications 1 à 6, le kit de test comprenant au moins un anticorps anti FLC et au moins un anticorps anti antigène de cancer LCBOP.
